(19)

Europäisches
Patentamt
European
Patent Office
Office européen
des brevets

(11) **EP 2 260 809 A1**

(12) **EUROPEAN PATENT APPLICATION**
published in accordance with Art. 153(4) EPC

(43) Date of publication:
**15.12.2010 Bulletin 2010/50**

(21) Application number: **09727217.3**

(22) Date of filing: **19.02.2009**

(51) Int Cl.:
*A61F 13/15* (2006.01)    *A61F 13/494* (2006.01)

(86) International application number:
**PCT/JP2009/052840**

(87) International publication number:
**WO 2009/122804 (08.10.2009 Gazette 2009/41)**

(84) Designated Contracting States:
**AT BE BG CH CY CZ DE DK EE ES FI FR GB GR
HR HU IE IS IT LI LT LU LV MC MK MT NL NO PL
PT RO SE SI SK TR**
Designated Extension States:
**AL BA RS**

(30) Priority: **31.03.2008 JP 2008093139**

(71) Applicant: **Uni-charm Corporation
Ehime 799-0111 (JP)**

(72) Inventors:
• **WAKASUGI, Kei
Kanonji-shi
Kagawa 769-1602 (JP)**

• **MISHIMA, Yoshitaka
Kanonji-shi
Kagawa 769-1602 (JP)**
• **KIKUCHI, Kyo
Kanonji-shi
Kagawa 769-1602 (JP)**

(74) Representative: **Eke, Philippa Dianne
Saunders & Dolleymore LLP
9 Rickmansworth Road
Watford
Hertfordshire WD18 0JU (GB)**

(54) **DISPOSAL DIAPER**

(57) The present invention aims to provide a disposable diaper including leak barriers improved in its function to prevent loose passage from infiltrating thereinto and eventually leaching out therethrough.

A disposable diaper (1) is provided with leak barriers (21) attached to opposite side edges of a crotch region (6) so that these leak barriers (21) may raise themselves on an inner sheet (3). The leak barriers (21) are formed of a nonwoven fabric made of thermoplastic synthetic fibers having a fineness in a range of 0.5 to 6dtex and have a specific volume in a range of 9 to 13cm$^3$/g and a thickness in a range of 0.35 to 0.75mm.

FIG.1

**Description**

TECHNICAL FIELD

The present invention relates to disposable diapers and more particularly to disposable diapers having an improved function to prevent loose passage from leaching out therethrough.

RELATED ART

In disposable diapers, it is well known to provide the crotch region with the leak barriers attached to its inner side of the crotch region along its opposite side edges so that these leak barriers can be spaced from the inner side of the crotch region. As the measure to improve the leak-barrier efficiency of these barriers, for example, JP 3761896 B1 (PATENT DOCUMENT 1) discloses the invention according to which the hydraulic resistance of these leak barriers is improved by laminating melt blown fibrous layer and spun bonded fibrous layer one on another.
PATENT DOCUMENT 1: JP 3761896 B1

DISCLOSURE OF THE INVENTION

PROBLEMS TO BE SOLVED BY THE INVENTION

However, the melt blown fibrous layer is generally formed of densely piled up ultrafine fibers and apt to improve the hydraulic pressure resistance at a cost of breathability of the leak barriers. Furthermore, loose passage generally has a surface tension sufficiently low to infiltrate easily into voids left among thermoplastic synthetic fibers densely piled up in the leak barriers and loose passage might leak out through the barriers.

In view of the problems as has been described above, it is a principal object of the present invention to provide a disposable diaper including leak barriers improved in its function to prevent loose passage from infiltrating thereinto and eventually leaching out therethrough.

MEASURE TO SOLVE THE PROBLEMS

The object set forth above is achieved, according to the present invention, by an improvement in a disposable diaper having a crotch region, a front waist region extending forward from the crotch region and a rear waist region extending rearward from the crotch region and comprising a liquid-pervious inner sheet, a liquid-impervious outer sheet and a bodily fluid-absorbent structure inclusive of a bodily fluid-absorbent core sandwiched between the inner and outer sheets in the crotch region wherein the crotch region is provided on its inner side along opposite side edges thereof with leak barriers formed of a nonwoven fabric extending across the crotch region further into the front and rear waist regions and adapted to raise themselves on the inner sheet toward diaper wearer's skin.

The improvement according to the present invention is **characterized in that** the nonwoven fabric is formed of thermoplastic synthetic fibers having a fineness in a range of 0.5 to 6dtex and has a specific volume in a range of 9 to 13cm$^3$/g and a thickness in a range of 0.35 to 0.75mm.

EFFECT OF THE INVENTION

In the disposable diaper according to the present invention, the specific volume and the thickness of the nonwoven fabric used to form the leak barriers are appropriately selected and combined with each other to prevent loose passage from leaching out through the leak barriers and at the same time to improve breathability thereof. In this way, the leak-barrier function of the diaper as a whole is improved.

BRIEF DESCRIPTION OF THE ACCOMPANYING DRAWINGS

[FIG. 1] Fig. 1 is a partially cutaway perspective view of a disposable diaper according to the present invention.
[FIG. 2] Fig. 2 is a sectional view taken along the line II-II in Fig. 1.

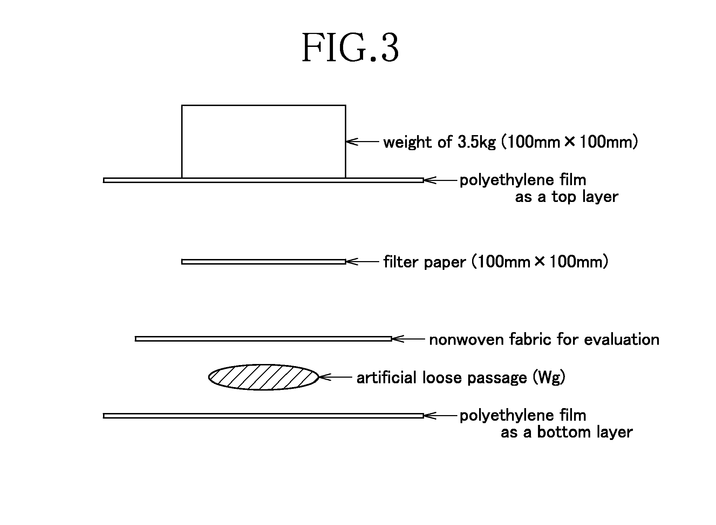

[FIG. 3] Fig. 3 is a schematic diagram illustrating a method to measure a leach rate.
[FIG. 4] Fig. 4 is a graphic diagram plotting a measurement result scored on TABLE 1.

IDENTIFICATION OF REFERENCE NUMERALS USED IN THE DRAWINGS

| 1 | disposable diaper |
|---|---|
| 2 | inner sheet |
| 3 | outer sheet |
| 4 | core |
| 5 | bodily fluid-absorbent structure |
| 6 | crotch region |
| 7 | front waist region |
| 8 | rear waist region |
| 21 | leak barrier |

DETAILED DESCRIPTION OF THE PREFERRED EMBODIMENTS

Details of the disposable diaper according to the present invention will be more fully understood from the description given hereunder with reference to the accompanying drawings.

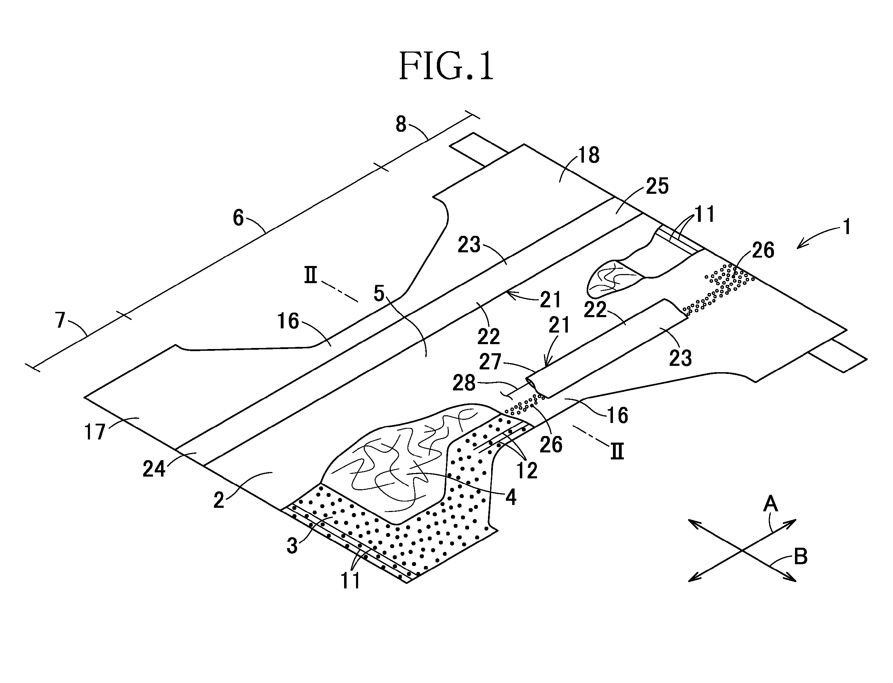

Fig. 1 is a partially cutaway perspective view of the disposable diaper 1. The diaper 1 comprises a liquid-pervious inner sheet 2, a liquid-impervious outer sheet 3 and a bodily fluid-absorbent core 4 wherein the diaper 1 has a crotch region 6, a front waist region 7 defined before the crotch region 6 and a rear waist region 8 defined behind the crotch region 6. Of the crotch region 6, the front waist region 7 and the rear waist region 8, at least the crotch region 6 is occupied by a bodily fluid-absorbent structure 5 formed of the inner and outer sheets 2, 3 and the core 4. The rear waist region 8 is provided on opposite side edges thereof with tape fasteners 13 used to join the front and rear waist regions 7, 8 to each other. The inner and outer sheets 7, 8 extend outward from a peripheral edge of the core 4 and are put flat and bonded together by hot melt adhesive 14 outside the peripheral edge of the core 4 to form a pair of side flaps 16, a front end flap 17 and a rear end flap 18. The front end flap 17 and the rear end flap 18 are respectively provided with waist elastic members 11 attached under tension between the inner sheet 2 and the outer sheet 3 and extending in a transverse direction B of the diaper 1. Segments of the side flaps 16 extending in the crotch region 6 are respective provided with leg elastic members 12 attached under tension between the inner sheet 2 and the outer sheet 3 and extending in a front-back direction A of the diaper 1. On an inner side of the diaper 1, the crotch region 6 is provided along opposite side edges thereof with leak barriers 21, respectively. These leak barriers 21 extend across the crotch region 6 further into the front waist region 7 and the rear waist regions 8. Each of these leak barriers 21 has an inner lateral portion 22, an outer lateral portion 23, a front end portion 24 and a rear end portion 25 wherein the outer lateral portion 23, the front end portion 24 and the rear end portion 25 are attached to the inner sheet 2 by hot melt adhesive 26. The inner lateral portion 22 has its outer end folded to form a sleeve 27 within which an elastic member 28 extend in the front-back direction A under tension. Specifically, at least opposite ends (not shown) of the elastic member 28 are fixed to the inner surface of the sleeve 27 by adhesive.

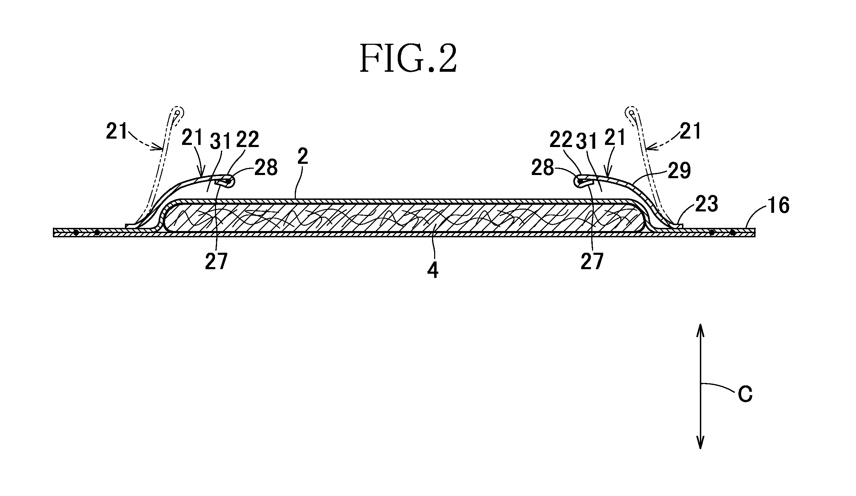

Fig. 2 is a sectional view taken along the line II-II in Fig. 1. Each of the leak barriers 21 partially attached to the inner side of the diaper 1 in the manner as has been described above has its inner lateral portion 22 lying above the core 4 as viewed in a thickness direction C of the core 4 and adapted to be spaced upward from the inner sheet 2. The outer lateral portion 23 is attached to the inner side of the associated side flap 16. Each of the leak barriers 21 additionally has an intermediate portion 29 defined between the inner lateral portion 22 and the outer lateral portion 23. The intermediate portion 29 cooperates with the inner sheet 2 to form therebetween a pocket 31 adapted to receive body waste and to retain this.

The elastic members 28 for the leak barriers 21 contract as the diaper 1 is put on the wearer's body and curved in the front-back direction A. Thereupon the inner lateral portions 22 and the intermediate portions 29 raise themselves on the inner sheet 2 as indicated by imaginary lines in Fig. 2. The respective inner lateral portions 22 of the leak barriers 21 raising themselves in this manner come in contact with legs and inguinal region of the wearer (not shown) and thereby prevent bodily fluids from leaking sideways out of the diaper 1.

In such diaper 1, the inner sheet 2 may be formed by liquid-pervious nonwoven fabric and the outer sheet 3 may be formed of a plastic film, a nonwoven fabric or a composite sheet consisting of a plastic film and a nonwoven fabric. The core 4 may be formed by wrapping fluff pulp fibers and/or super-absorbent polymer particles with a tissue paper. The leak barriers 21 may be formed of a nonwoven fabric.

With this diaper 1 put on the wearer's body, urine or loose passage flowing on the crotch region 6 in the transverse direction B is prevented by the leak barriers 21 from further flowing. In addition to such primary function, the leak barriers 21 preferably assures an improved breathability and fulfills the other function to prevent loose passage from leaching out of the leak barriers 21. It should be noted here that a surface tension of loose passage is lower than that of urine and therefore may easily infiltrate into fiber interstices of the nonwoven fabric forming the leak barriers 21. In consideration of this, the nonwoven fabric to be used as stock material for the leak barriers 21 should be evaluated and selected from the standpoint other than a function of these barriers for blocking undesirable flow of urine.

TABLE 1 summarizes the results of evaluation tests having been conducted on various types of nonwoven fabrics to determine their propriety as stock material for the leak barriers 21 from the viewpoint as has been described above. In this evaluation test, a spun bonded nonwoven fabric (brevity code: SB or S), a melt blown nonwoven fabric (brevity code: MB or M) and an air-through nonwoven fabric (brevity code: AT) each formed of thermoplastic synthetic fibers having fineness in a range of 0.5 to 6dtex were combined in various manners to obtain a plurality of nonwoven fabric sheets for evaluation test which were different one from another in thickness and specific volume. On these nonwoven fabric sheets for evaluation test, leach rate of artificial loose passage and breathability were measured and the propriety was determined with respect to each of these nonwoven fabric sheets.

[TABLE 1]

| sample number | composition of nonwoven fabric | thickness (mm) | basis weight (g/m²) | specific volume (cm³/g) | leach rate (g/g) | breathability (cc/cm²* sec) |
|---|---|---|---|---|---|---|
| 1 | SB | 0.185 | 20 | 9.3 | 0.58 | 778 |
| 2 | SB | 0.403 | 40 | 10.1 | 0.40 | 389 |
| 3 | SB | 0.170 | 18 | 9.4 | 0.62 | 366 |
| 4 | SB | 0.308 | 36 | 8.6 | 0.52 | 189 |
| 5 | MB | 0.356 | 30 | 11.9 | 0.47 | |
| 6 | MB | 0.666 | 60 | 11.1 | 0.32 | |
| 7 | SB+MB | 0.268 | 28 | 9.6 | 0.51 | |
| 8 | SB+MB | 0.526 | 56 | 9.4 | 0.35 | |
| 9 | SMS | 0.790 | 54 | 14.6 | 0.28 | 72 |
| 10 | SMS | 1.420 | 104 | 13.7 | 0.02 | 33 |
| 11 | SMS | 0.510 | 29 | 17.6 | 0.45 | 105 |
| 12 | SMS | 0.850 | 48 | 17.7 | 0.13 | 45 |
| 13 | SMS | 0.800 | 57 | 14.0 | 0.20 | 60 |
| 14 | SMS | 1.350 | 114 | 11.8 | 0.01 | 27 |
| 15 | SMS | 0.450 | 32 | 14.1 | 0.40 | 63 |
| 16 | SMS | 0.820 | 64 | 12.8 | 0.12 | 30 |
| 17 | SMS | 0.830 | 60 | 13.8 | 0.20 | 35 |
| 18 | SMS | 1.450 | 120 | 12.1 | 0.00 | 17 |
| 19 | SMS | 0.460 | 35 | 13.1 | 0.36 | 42 |
| 20 | SMS | 0.850 | 70 | 12.1 | 0.04 | 20 |
| 21 | SB | 0.350 | 25 | 14.0 | 0.52 | 673 |
| 22 | SB | 0.600 | 50 | 12.0 | 0.35 | 276 |
| 23 | SMS | 0.186 | 17 | 10.9 | 0.58 | 194 |
| 24 | SMS | 0.24 | 26 | 9.2 | 0.54 | 97 |
| 25 | AT | 0.734 | 40 | 18.4 | 0.32 | 803 |
| 26 | AT | 0.718 | 50 | 14.4 | 0.33 | 684 |

A method of measuring their leach rate, one of evaluation items in TABLE 1, is schematically illustrated by Fig.

3 and comprises steps of:

(1) mixing commercially available mayonnaise (made by Q.P. Corporation) with tap water at ratio of 4:1 to produce artificial loose passage,
(2) collecting about 1g of artificial loose passage and accurately weighing (Wg) and dropped it onto a middle of a polyethylene film as a low layer,
(3) weighing filter paper of 100 x 100mm (Ag),
(4) stacking each 150 x 150mm of the nonwoven fabric sheet for evaluation, the filter paper and the polyethylene film as a top layer in the order as illustrated on artificial loose passage,
(5) placing a weight having a bottom surface area of 100 x 100mm and weighing 3.5kg on the polyethylene film as the top layer and left to stand for 30sec,
(6) Removing the weight and weighing the filter paper (Bg), and
(7) calculating a leach rate by the following equation.

[Equation 1]

$$\text{leach rate (g/g)} = \frac{B-A}{W}$$

To measure their breathability, one of the evaluation items indicated in TABLE 1, a breathability resistance value (kPa·s/m) was measured and then the breathablity was calculated from the following equation.
[Equation 2]

$$\text{breathability (cc/cm}^2\text{*sec)} = \frac{124.55}{\text{aeration resistance} \times 1000} \times 100$$

On the basis of the leach rate and the breathability measured in the above-mentioned manner, the inventors have found that the nonwoven fabric sheet having a leach rate of 0.50g/g or less can be determined to be appropriate as stock material for the leak barriers 21 and this leach rate value of 0.50g/g can be employed as the reference value for determination of the propriety of the leach out preventing ability. With respect to the breathability, the nonwoven fabric sheet having a breathability of 180cc/cm$^2$*sec or higher can be determined to be appropriate for use as the leak barriers 21 since such nonwoven fabric sheet will be substantially free from a problem of stuffiness. Based on such findings, the inventors employed this value as the reference value for determination of the propriety of the breathability.

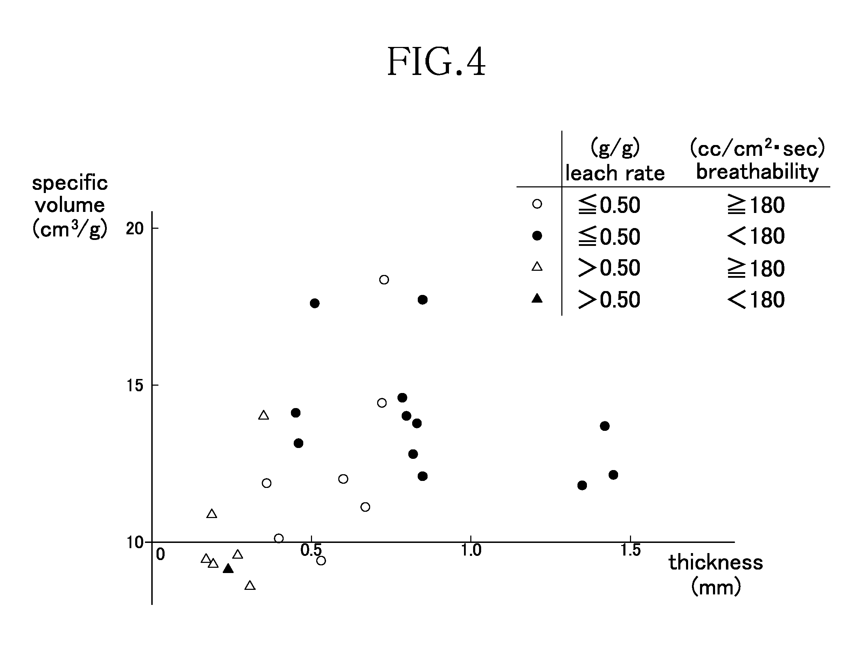

In Fig. 4, a white circle indicates that the leach rate does not exceed its reference value and the breathability is not less than its reference value and therefore the nonwoven fabric sheet is appropriate for use as the leak barriers. A black circle indicates that the leach rate does not exceed the reference value but the breathability is lower than the reference value and therefore the nonwoven fabric sheet is not appropriate for used as the leak barriers. A white triangle indicates that the breathability is not less than the reference value but the leach rate exceeds the reference value and therefore the nonwoven fabric sheet is not appropriate for use as the leak barriers. A black triangle indicates that the leach rate exceeds the reference value and the breathability is lower than the reference value and therefore the nonwoven fabric sheet is not appropriate for use as the leak barriers.
As will be apparent from TABLE 1 and Fig. 4, the nonwoven fabric sheet having a thickness in a range of 0.35 to 0.75mm and a specific volume in a range of 9 to 13cm$^3$/g is appropriate as stock material for the leak barriers 21 since such nonwoven fabric sheet has a sufficiently small leach rate and a sufficiently high breathability.

**Claims**

1. A disposable diaper having a crotch region, a front waist region extending forward from said crotch region and a rear waist region extending rearward from said crotch region and comprising a liquid-pervious inner sheet, a liquid-impervious outer sheet and a bodily fluid-absorbent structure inclusive of a bodily fluid-absorbent core sandwiched between said inner and outer sheets in said crotch region wherein said crotch region is provided on its inner side along opposite side edges thereof with leak barriers formed of a nonwoven fabric extending across said crotch region further into said front and rear waist regions and adapted to raise themselves on said inner sheet toward diaper wearer's skin, said disposable diaper being **characterized in that**:

   said nonwoven fabric is made of thermoplastic synthetic fibers having a fineness in a range of 0.5 to 6dtex and has a specific volume in a range of 9 to 13cm$^3$/g and a thickness in a range of 0.35 to 0.75mm.

# FIG.1

EP 2 260 809 A1

# FIG.2

EP 2 260 809 A1

# FIG.3

weight of 3.5kg (100mm × 100mm)

polyethylene film
as a top layer

filter paper (100mm × 100mm)

nonwoven fabric for evaluation

artificial loose passage (Wg)

polyethylene film
as a bottom layer

EP 2 260 809 A1

# FIG.4

## INTERNATIONAL SEARCH REPORT

International application No.

PCT/JP2009/052840

A.  CLASSIFICATION OF SUBJECT MATTER
*A61F13/15(2006.01)i, A61F13/494(2006.01)i*

According to International Patent Classification (IPC) or to both national classification and IPC

B.  FIELDS SEARCHED

Minimum documentation searched (classification system followed by classification symbols)
A61F13/15, A61F13/494

Documentation searched other than minimum documentation to the extent that such documents are included in the fields searched
Jitsuyo Shinan Koho          1922-1996    Jitsuyo Shinan Toroku Koho    1996-2009
Kokai Jitsuyo Shinan Koho    1971-2009    Toroku Jitsuyo Shinan Koho    1994-2009

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y | JP 10-314218 A  (Kuraray Co., Ltd.), 02 December, 1998 (02.12.98), Full text; all drawings (Family: none) | 1 |
| Y | CD-ROM of the specification and drawings annexed to the request of Japanese Utility Model Application No. 56458/1992 (Laid-open No. 17724/1994) (Uni-Charm Corp.), 08 March, 1994 (08.03.94), Par. No. [0012]; Figs. 1, 2 (Family: none) | 1 |

☒   Further documents are listed in the continuation of Box C.         ☐   See patent family annex.

| | |
|---|---|
| * Special categories of cited documents:<br>"A"  document defining the general state of the art which is not considered   to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| 19 May, 2009 (19.05.09) | 02 June, 2009 (02.06.09) |

| Name and mailing address of the ISA/<br>Japanese Patent Office | Authorized officer |
|---|---|
| Facsimile No. | Telephone No. |

Form PCT/ISA/210 (second sheet) (April 2007)

| | **INTERNATIONAL SEARCH REPORT** | International application No. |
|---|---|---|
| | | PCT/JP2009/052840 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 3761896 B2  (The Procter & Gamble Co.),<br>29 March, 2006 (29.03.06),<br>Full text; all drawings<br>& US 5624425 A          & EP 818979 A<br>& WO 1996/031176 A1      & DE 69610416 T | 1 |
| A | JP 2002-35032 A  (Daio Paper Corp.),<br>05 February, 2002 (05.02.02),<br>Claims; Figs. 1, 2<br>(Family: none) | 1 |
| A | JP 2000-254171 A  (Kao Corp.),<br>19 September, 2000 (19.09.00),<br>Claims; all drawings<br>& US 6706030 B1          & EP 1164998 A<br>& WO 2000/053140 A1      & DE 60020536 T | 1 |

Form PCT/ISA/210 (continuation of second sheet) (April 2007)

**EP 2 260 809 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3761896 B